# EUROPÄISCHE PATENTSCHRIFT

(11) **EP 2 347 724 B1**
(45) Veröffentlichungstag und Bekanntmachung des Hinweises auf die Patenterteilung: **21.08.2019**
(21) Anmeldenummer: 11000391.0
(22) Anmeldetag: 19.01.2011
(51) Int. Cl.: A61B 17/16, A61B 17/88, B23B 31/20, B25B 23/10, B25B 23/00

(54) **Spannvorrichtung für chirurgische Elemente**
Tensioning device for surgical elements
Dispositif de serrage pour éléments chirurgicaux

(30) Priorität: 26.01.2010 DE 102010005706
(43) Veröffentlichungstag der Anmeldung: 27.07.2011
(73) Patentinhaber: Stryker European Holdings I, LLC, Kalamazoo, MI 49002 (US)
(72) Erfinder: Cremer, Axel, 3428 Wiler (CH); Aebi, This, 2540 Grenchen (CH)
(74) Vertreter: Röthinger, Rainer

(56) Entgegenhaltungen:
- DE-A1- 4 103 663
- DE-C1- 19 650 799
- DE-U1- 9 214 609
- US-A- 633 852
- US-A- 2 701 494
- US-A- 2 878 701
- US-A- 4 936 843
- US-A1- 2007 171 540
- US-A1- 2008 246 233

## Beschreibung

### Technisches Gebiet

Chirurgische Elemente wie Knochenschrauben werden u.a. bei externen Fixationssystemen verwendet. Um die Schrauben in einen Knochen einzubringen sind aktuell für unterschiedliche Schrauben-Durchmesser unterschiedliche Werkzeug-Kupplungen mit geeigneten Spannvorrichtungen zu verwenden. Dies ist insofern mühsam, als durchaus in einem gegebenen Fixationsrahmen unterschiedliche Schrauben-Durchmesser verwendet werden (z.B. Knee-Bridging - im Femur: Schrauben mit 6 mm, in der Tibia: Schrauben mit 5 mm).

Die aktuellen Kupplungen müssen von Hand geöffnet und geschlossen werden. Trotzdem ist häufig ein merkliches Spiel der Schrauben in der Spannvorrichtung vorhanden. Dies führt vor allem beim Antrieb mittels eines Elektrowerkzeugs zu unerwünschtem Schlingern in der Luft.

Beispielsweise zeigt die DE 10 2005 058 868 A1 einen Schraubendreher für Knochenschrauben, der ein Griffteil und einen daran drehfest gehaltenen Schaft aufweist. Das freie Ende des Schafts weist einen unrunden Querschnitt auf und ist formschlüssig in eine Aufnahmeöffnung in einem Kopf einer Knochenschraube einsetzbar. Um das freie Ende des Schafts in die Aufnahmeöffnung der Knochenschraube einzusetzen, muss eine Griffhülse axial verschoben werden, wodurch eine Schraubenfeder gespannt wird. Dabei wird ferner ein Stab in einer Längsnut des Schafts in Richtung auf das Griffteil verschoben und vollständig in der Längsnut aufgenommen. Nachdem der Schaft in die Aufnahmeöffnung eingeführt wurde, wird die Griffhülse durch einen Operateur wieder freigegeben, so dass sich unter der Wirkung der sich entspannenden Schraubenfeder der Stab in Richtung auf das freie Ende verschiebt und aufgrund einer Aufgleitfläche radial nach außen gegen die Innenwand der Aufnahmeöffnung gedrückt wird.

Die DE 199 45 322 A1 beschreibt ein Spannfutter für ein chirurgisches Bohrgerät. Zum Einführen eines Bohrdrahts in das Spannfutter wird dieser durch eine Bohrung in der Endfläche eines Führungskörpers hindurchgesteckt und weiter in eine Durchgangsbohrung einer Backenführung hineingeführt. Der Bohrdraht stößt dann an Aufgleitflächen von durch eine Blattfeder vorgespannten Klemmbacken an und spreizt dadurch die Klemmbacken in radialer Richtung nach außen. Die vorgespannten Klemmbacken werden ferner soweit in axialer Richtung mitgenommen, bis sie mit ihrem Backenende an einem Stützelementvorsprung anstoßen. In einer Endstellung liegen dann die Klemmflächen der Klemmbacken vollständig am Bohrdraht an, so dass es zu einer Verklemmung zwischen den Klemmbacken und dem Bohrdraht kommt.

Aus den Druckschriften US 2008/0246233 A1, DE 41 03 663 A1 und US 2007/0171540 A1 ist jeweils eine Spannvorrichtung für Werkzeugelemente bekannt, die wenigstens einen kugelförmigen Klemmkörper umfasst, welcher bei einem Einspannvorgang entlang einer geneigten Spannfläche abrollt und dabei eine zur Aufnahme von Werkzeugelementen vorgesehene Längsbohrung verengt.

Aus der Druckschrift DE 196 50 799 C1 A1 ist ferner ein Schraubvorsatz mit einer hülsenförmigen Halterung und einer vorgespannten Nuss bekannt. Die Nuss besteht aus zwei Backen die bei einer entgegen der Vorspannrichtung anliegenden externen Kraft in die Halterung gedrückt werden. Die Backen werden dabei aufgrund von an der Halterung und an den Backen vorgesehenen, spitzwinklig verlaufenden Auflaufflächen in radialer Richtung zusammengedrückt.

### Kurzer Abriss

Die hier vorgeschlagene Spannvorrichtung für chirurgische Elemente umfasst eine Fixiereinrichtung, die zur drehfesten Fixierung eines chirurgischen Elements ausgebildet ist, eine Klemmeinrichtung, die zur Ausübung einer im Wesentlichen radial nach innen auf das chirurgische Element wirkende Klemmkraft ausgebildet ist, eine Federeinrichtung, welche zur Ausübung einer Federkraft auf die Klemmeinrichtung ausgebildet ist, wobei die Federkraft in axialer Richtung wirkt, und eine mit der Klemmeinrichtung zusammenwirkende Kraftumlenkeinrichtung, welche ausgebildet ist, zumindest einen Teil der auf die Klemmeinrichtung wirkenden Federkraft in zumindest einen Teil der auf das chirurgische Element wirkenden Klemmkraft umzuwandeln. Klemmeinrichtung und Fixiereinrichtung können als getrennte Bauteile realisiert sein oder in einem einzigen Bauteil integriert werden.

Die Klemmeinrichtung kann eine Kontaktierungsfläche für das chirurgische Element aufweisen, die bei einer Einführung des chirurgischen Elements in die Spannvorrichtung bewirkt, dass die Klemmeinrichtung entgegen der Federkraft verschoben wird.

Auf diese Weise lässt sich eine Klemmkraft aufbringen oder erhöhen. Eine Erhöhung der Klemmkraft ist beispielsweise dann möglich, wenn die Klemmeinrichtung bereits von sich aus einen Klemmkraftanteil bereit stellt.

Insgesamt kann die Klemmeinrichtung einen im Wesentlichen rotationssymmetrischen Aufbau besitzen. Die Klemmeinrichtung umfasst einen oder mehrere auslenkbare Klemmfinger. Sofern mehrere Klemmfinger vorhanden sind, können diese das chirurgische Element in Umfangsrichtung umgeben. Die Klemmfinger können dabei in Umfangsrichtung jeweils den gleichen Abstand voneinander aufweisen.

Der wenigstens eine Klemmfinger kann eine Schrägfläche aufweisen, die bei einem Zusammenwirken mit dem chirurgischen Element eine Auslenkung des Klemmfingers radial nach außen bewirkt. Die Schrägfläche kann mit der oben erwähnten Kontaktierungsfläche übereinstimmen.

Die Kraftumlenkeinrichtung kann eine erste Schrägfläche umfassen, gegen welche die Klemmeinrichtung mittels der Federeinrichtung vorgespannt oder vorspannbar ist. Es können mehrere (beispielsweise voneinander beabstandete) erste Schrägflächen vorgesehen werden. Ferner ist es denkbar, dass die erste Schrägfläche eine rotationssymmetrische und/oder konische Form aufweist. Sofern die Klemmeinrichtung ebenfalls eine rotationssymmetrische Form aufweist oder das chirurgische Element zumindest in Umfangsrichtung umgibt, kann das Element spielfrei stabilisiert werden, so dass das bekannte Schlingern vermieden werden kann.

Die Klemmeinrichtung kann wenigstens eine zweite Schrägfläche umfassen, die mit der wenigstens einer ersten Schrägfläche der Kraftumlenkeinrichtung in kraftumlenkender Weise zusammenwirkt. So wäre es beispielsweise denkbar, dass jeweils eine zweite Schrägfläche an jeweils einem Klemmfinger vorgesehen ist. Für den Fachmann liegt es auf der Hand, dass für die Kraftumlenkfunktion prinzipiell eine einzige Schrägfläche ausreichend ist. So kann beispielsweise lediglich die erste Schrägfläche der Kraftumlenkvorrichtung vorgesehen sein, und die zweite Schrägfläche im Bereich der Klemmeinrichtung durch eine anders geformte Kontaktierungsfläche oder sogar durch einen linienförmigen Kontaktbereich ersetzt werden könnte.

Die Fixiereinrichtung kann eine Öffnung zur Aufnahme eines Teils des chirurgischen Elements aufweisen. Beispielsweise kann ein distaler Teil des chirurgischen Elements in der Öffnung der Fixiereinrichtung aufgenommen werden. Die Begriffe distal bzw. proximal beziehen sich im Rahmen der vorliegenden Offenbarung auf eine Richtung von der Operationsstelle weg bzw. auf die Operationsstelle zu.

Die Öffnung der Fixiereinrichtung kann mit einer Profilierung versehen sein, die eine drehfeste Kopplung mit dem eine komplementäre Profilierung aufweisenden chirurgischen Element gestattet. Die Profilierung kann beispielsweise die Gestalt eines Mehrkant-Profils annehmen.

Auch ist es denkbar, dass die Öffnung der Fixiereinrichtung mehrere in axialer Richtung voneinander beabstandete und sich in distaler Richtung (z.B. stufenförmig) verjüngende Abschnitte aufweist. Eine derartige Ausgestaltung der Öffnung ermöglicht die drehfeste Aufnahme von chirurgischen Elementen unterschiedlichen Außendurchmessers. Generell können die Außendurchmesser der chirurgischen Elemente im Millimeterbereich (z.B. von ungefähr 1 bis ungefähr 10 mm) liegen.

Die Spannvorrichtung kann ferner ein Gehäuseteil umfassen, welches die Klemmeinrichtung außen wenigstens teilweise umgibt. Die Kraftumlenkeinrichtung kann an dem Gehäuseteil (beispielsweise an einem distalen Abschnitt des Gehäuseteils) ausgebildet sein. Ferner kann am Gehäuseteil ein Anschlag ausgebildet sein, gegen den die Klemmeinrichtung im Ausgangszustand federnd vorgespannt oder vorspannbar ist.

Des Weiteren kann die Spannvorrichtung einen Grundkörper umfassen, in dem die Fixiereinrichtung ausgebildet ist. Am Grundkörper kann ferner ein Gegenlager für die Federeinrichtung ausgebildet sein. Zusätzlich oder alternativ hierzu kann das Gehäuseteil mit dem Grundkörper gekoppelt sein. Die Kopplung kann derart erfolgen, dass eine vorgegebene oder vorgebbare axiale Relativposition zwischen dem Gehäuseteil und dem Grundkörper gewährleistet ist. Diese axiale Relativposition kann die Vorspannung der Klemmeinrichtung (und damit die Klemmkraft) im Ausgangszustand, also vor dem Aufnehmen eines chirurgischen Elements, definieren. Geeignete Koppelmechanismen beinhalten beispielsweise Schraubverbindungen, Rastverbindungen, und dergleichen.

Gemäß einer Weiterbildung kann am Grundkörper eine Koppeleinrichtung zur Kopplung mit einem chirurgischen Werkzeug oder einem Werkzeughandgriff vorgesehen sein. Bei dem chirurgischen Werkzeug kann es sich um ein chirurgisches Elektrowerkzeug (wie beispielsweise einen batteriebetriebenen Schrauber) handeln.

Ebenfalls vorgeschlagen wird ein chirurgisches Werkzeug, welches die Spannvorrichtung umfasst. Außerdem wird ein chirurgisches System angegeben, welches dieses chirurgische Werkzeug sowie mehrere einzuspannende chirurgische Elemente mit unterschiedlichen Außendurchmessern umfasst. Die chirurgischen Elemente können beispielsweise Knochenschrauben, gewindelose Knochenpins, Bohrer, KirschnerDrähte (mit und ohne Gewinde) usw. umfassen.

Das chirurgische System kann ferner wenigstens ein Rahmenelement (zum Beispiel eine Stange) eines externen Fixateurs beinhalten. Die chirurgischen Elemente können in diesem Fall zur Befestigung des wenigstens einen Rahmenelements an einem Knochen ausgebildet sein.

### Beschreibung der Zeichnungen

Weitere Einzelheiten, Vorteile und Aspekte der hier beschriebenen Spannvorrichtung für chirurgische Elemente ergeben sich aus den nachfolgenden Zeichnungen. Es zeigen:
- Fig. 1: eine Explosionsdarstellung eines Ausführungsbeispiels einer Spannvorrichtung für chirurgische Elemente;
- Fig. 2: eine Schnittansicht der Spannvorrichtung für chirurgische Elemente im Ausgangszustand gemäß Fig. 1; und
- Fig. 3: eine Schnittansicht der Spannvorrichtung für chirurgische Elemente gemäß Fig. 1, wobei ein chirurgisches Element in die Spannvorrichtung eingespannt ist.

### Detaillierte Beschreibung

Im Folgenden wird ein Ausführungsbeispiel einer Spannvorrichtung für chirurgische Elemente erläutert. Übereinstimmende Elemente in den Figuren sind dabei mit denselben Bezugszeichen bezeichnet. Die Angaben "Vorderseite" und "Rückseite" beziehen sich auf die in den Figuren dargestellte Ausrichtung der Spannvorrichtung. Es versteht sich, dass die Spannvorrichtung im Betrieb beliebig ausgerichtet sein kann.

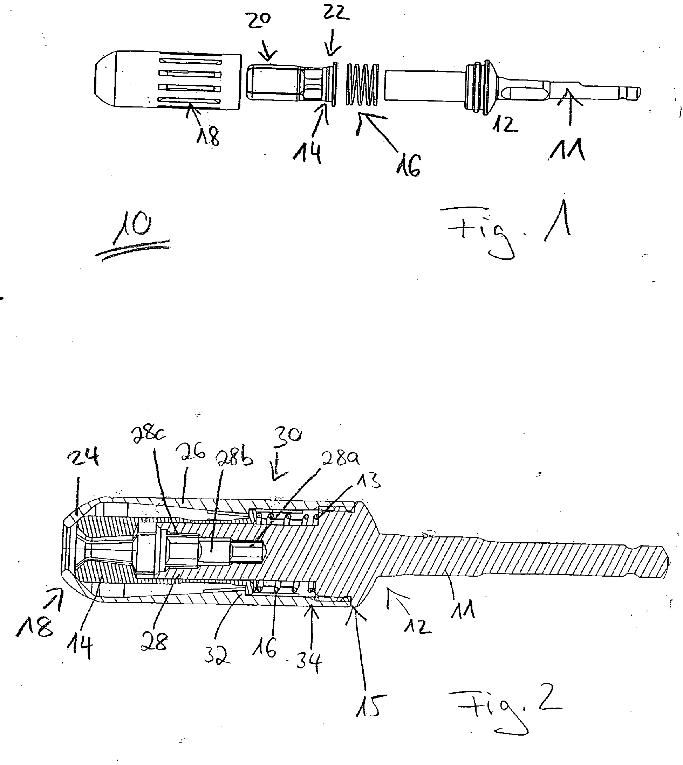

Fig. 1 zeigt in einer Explosionsdarstellung wesentliche Bauteile einer Spannvorrichtung 10 zur Einspannung von chirurgischen Elementen wie beispielsweise Knochenschrauben, Knochenbohrer oder Kirschnerdrähte. Die Spannvorrichtung 10 umfasst einen Grundkörper 12, eine Klemmeinrichtung 14, eine Federeinrichtung 16 sowie eine als Gehäuseteil ausgebildete Kraftumlenkeinrichtung 18. Diese Bauteile sind jeweils rotationssymmetrisch ausgebildet.

Der Grundkörper 12 ist als einstückige, zylinderförmige Welle ausgebildet, welche vorderseitig für die Aufnahme der Klemmeinrichtung 14, der Federeinrichtung 16 sowie der chirurgischen Elemente und rückseitig zur Kopplung mit einem chirurgischen (Elektro-)Werkzeug vorgesehen ist. Hierfür weist der hintere Abschnitt der Welle 11 flächenhafte Segmente für die drehfeste Ankopplung an das chirurgische Werkzeug auf, welches eine Rotationsbewegung auf die Welle überträgt.

Wie in der Schnittansicht gemäß Fig. 2 erkennbar, ist am Grundkörper 12 etwa mittig ein rotationssymmetrischer Überstand 15 ausgebildet, welcher an seiner vorderen Stirnfläche ein Gegenlager 13 für die Federeinrichtung 16 ausbildet. Die Federeinrichtung 16 ist im Ausführungsbeispiel als Schraubenfeder ausgebildet, welche passgenau über den vorderen Abschnitt des Grundkörpers 12 gestülpt wird und nach hinten bis zum Anschlag am Gegenlager 13 geschoben wird.

Die Klemmeinrichtung 14 ist als einstückige, hohlzylinderförmige Spannzange ausgebildet, wobei die Spannzange vier Klemmfinger 20 aufweist (in Fig. 1 sind nur drei Klemmfinger aufgrund der perspektivischen Darstellung sichtbar), wobei die vier Klemmfinger 20 jeweils über einen schmalen Steg mit einem hohlzylinderförmigen Fußelement 22 verbunden sind. Die Klemmeinrichtung 14 wird im Anschluss an die Schraubenfeder 16 über den vorderen Abschnitt des Grundkörpers 12 gestülpt, bis sie in Anlage an die Schraubenfeder 16 gelangt. Dadurch kann die Schraubenfeder 16 zwischen dem Fußelement 22 der Klemmeinrichtung 14 und dem Gegenlager 13 des Grundkörpers 12 eingespannt werden.

Die als Gehäuseteil ausgebildete Kraftumlenkeinrichtung 18 ist zylinderförmig ausgebildet, wobei sich die Kraftumlenkeinrichtung 18 zur Vorderseite hin konisch verjüngt. Die Kraftumlenkeinrichtung 18 besitzt je eine Öffnung an ihrer Vorder- und Rückseite sowie einen Hohlraum im Inneren zur Aufnahme der Klemmeinrichtung 14 samt Federeinrichtung 16. Bei der Montage der Spannvorrichtung 10 wird die Kraftumlenkeinrichtung 18 über die am vorderen Bereich des Grundkörpers 12 montierte Schraubenfeder 16 und Klemmeinrichtung 14 gestülpt. Anschließend wird die Kraftumlenkeinrichtung 18 an ihrem hinteren Ende mittels einer Schraubverbindung am Überstand 15 befestigt.

Die Kraftumlenkeinrichtung 18 bildet zusammen mit dem stufenförmigen Überstand 15 des Grundkörpers 12 ein schützendes Gehäuse der Spannvorrichtung 10, wobei der Überstand 15 einen Gehäuseboden und die Kraftumlenkeinrichtung 18 eine Mantel- und Deckelfläche darstellen.

Fig. 2 verdeutlicht das Zusammenwirken der einzelnen Bauteile. Eine wesentliche Bedeutung kommt dabei der Ausgestaltung der Innenflächen der Kraftumlenkeinrichtung 18 zu. Diese umfasst in ihrem Inneren vorderseitig eine konisch ausgebildete Schrägfläche 24, welche sich ringförmig und verjüngend bis zur vorderseitigen Öffnung der Kraftumlenkeinrichtung 18 erstreckt, und eine zweite Schrägfläche 26, welche direkt an die erste Schrägfläche 24 anschließt und sich konisch erweiternd auf Rückseite der Kraftumlenkeinrichtung 18 zuläuft. Die zweite Schrägfläche 26 endet in einer sich in radialer Richtung erstreckenden Abstufung 32, welche als Anlageelement für die in montierten Zustand in axialer Richtung bewegliche Klemmeinrichtung 14 dient. Die Klemmeinrichtung 14 wird in dem in Fig. 2 gezeigten Grundzustand (d.h. ohne Bestückung mittels eines chirurgischen Elements) von der Federeinrichtung 16 gegen die Abstufung vorgespannt. Die Vorspannkraft wird durch die axialen Relativpositionen von Grundkörper 12 und Umlenkeinrichtung 18 definiert.

Die Klemmeinrichtung 14 weist wie bereits erläutert vier Klemmfinger 20 auf, deren vorderseitigen Innenfläche jeweils zwei schräg zulaufende in Umfangsrichtung abgerundete Flächen umfassen. Die zwei schräg zulaufenden Flächen sind derart ausgebildet, dass die vier Klemmfinger 20, welche zueinander in Umfangsrichtung der Klemmeinrichtung 14 angeordnet sind, an ihrer Vorderseite eine doppelkonische bzw. doppeltrichterförmige Aufnahmeöffnung schaffen, in welche die chirurgischen Elemente zur Befestigung eingeschoben und zur Entnahme wieder herausgezogen werden. Die Klemmfinger 20 sind in radialer Richtung im Wesentlichen nach außen auslenkbar. Dabei zeichnen sich die Klemmfinger 20 durch eine kleine Federkonstante auszeichnen, um ein kraftsparendes Einschieben auch chirurgischer Elemente mit größerem Außendurchmesser zu gewährleisten. Dies wird durch geeignete Materialwahl der Klemmeinrichtung 14 erreicht.

Die jeweilige Außenseite der Klemmfinger 20 weist am vorderen Ende eine leicht angeschrägte Fläche auf, welche in axialer Richtung abgerundet in eine Außenfläche mündet, wobei die jeweiligen Außenseiten der Klemmfinger 20 zusätzlich in Umfangsrichtung abgerundet sind.

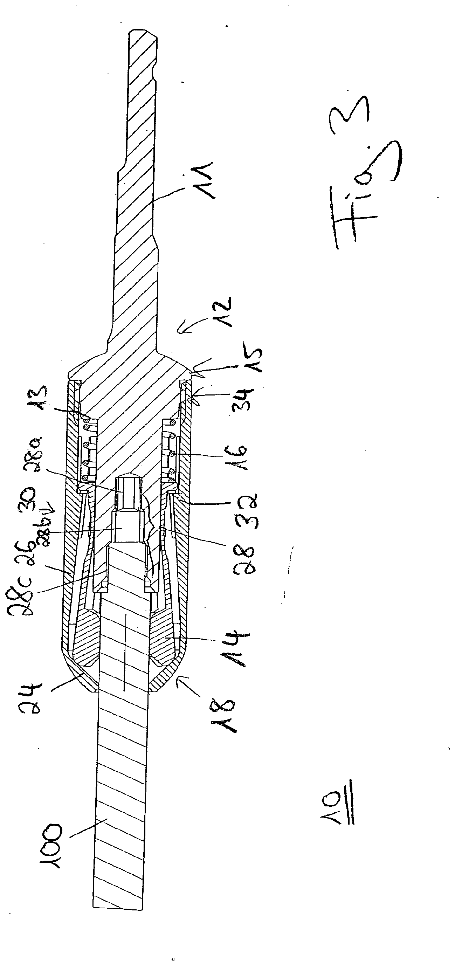

Der Grundkörper 12 umfasst an seinem vorderen Ende die Fixiereinrichtung 28, welche als konzentrisch angeordnete Aussparung in der zylinderförmigen Welle ausgebildet ist. Die Aussparung 18 umfasst dabei drei in axialer Richtung voneinander beabstandete und in distaler Richtung verjüngende Abschnitte, die, wie in Fig. 3 gezeigt, zur drehfesten Aufnahme eines chirurgischen Elementes 100 vorgesehen ist. Die drei Abschnitte weisen jeweils ein Vierkantprofil mit unterschiedlichen Durchmessern auf, in welches ein Befestigungsstift eines chirurgischen Elements mit entsprechenden Durchmesser mit komplementären Vierkantprofil drehmomentschlüssig eingesetzt werden.

Die Durchmesser der hier beschriebenen Fixiereinrichtung 28 sind für die Aufnahme von Schrauben mit Durchmessern von 4 mm, 5 mm und 6 mm ausgelegt. Es versteht sich, dass die vorliegende Offenbarung nicht auf die hier erwähnten Durchmesser beschränkt ist. Um Zugang zur Aussparung für alle Schraubengrößen zu gewähren, sind die Abschnitte der Aussparungen der Größe nach angeordnet, beginnend mit dem größten Durchmesser am vorderen Ende des Grundkörpers. Hierbei ist von Vorteil, dass die kleinste Durchmesser am weitesten hinten ausgebildet ist, da damit einer stabilere Aufnahme gewährleistet ist.

Im montierten Zustand befindet sich die Klemmeinrichtung 14 in der Spannvorrichtung 10 durch die Federeinrichtung 16 in axialer Richtung sowie durch die Kraftumlenkeinrichtung 18 in radialer Richtung vorgespannt. Die Einspannung in axialer Richtung durch die Federeinrichtung 16 wird dadurch bewirkt, dass die am vorderen Ende des Grundkörpers 12 eingesetzte Federeinrichtung 16 zwischen dem Gegenlager 13 des Grundkörpers 12 und dem Fußbereich der Klemmeinrichtung 14 zusammengedrückt wird. Dabei wird die axiale Beweglichkeit der Klemmeinrichtung 14 zur Vorderseite hin durch den Absatz 32 der Kraftumlenkeinrichtung 18 begrenzt.

Die radiale Vorspannung der Klemmfinger 20 wird hingegen durch die Führung bzw. Ablenkung der Klemmfinger 20 entlang der vorderseitigen Schrägfläche der Kraftumlenkeinrichtung 18 bewirkt. Somit umfasst die beschriebene Spannvorrichtung 10 im Wesentlichen zwei Spannmechanismen (Schraubenfeder einerseits und davon radial nach innen vorgespannte Klemmfinger 20 andererseits), welche miteinander seriell verknüpft sind.

Zum besseren Verständnis der Funktionsweise der hier beschriebenen Spannvorrichtung 10 zeigt Fig. 3 die mit dem chirurgischen Element 100 bestückte Spannvorrichtung 10. Das Element 100 wird durch die vorderseitige Öffnung in die Spannvorrichtung 10 manuell eingeführt, wobei durch den Durchmesser des chirurgischen Elements 100 (der größer ist als die durch die radial nach innen vorgespannten Klemmfinger 20 vorgegebene Öffnung) die Klemmfinger 20 axial nach hinten und radial nach außen gedrückt werden, wodurch die Klemmfinger 20 entlang der konischen Schrägfläche 24 der Kraftumlenkeinrichtung 18 geführt werden.

Die Klemmfinger 20 können sich nur öffnen, wenn gleichzeitig die gesamte Klemmeinrichtung 14 von der Einführbewegung in axialer Richtung gegen die Federkraft der Federeinrichtung 16 nach hinten verschoben wird. Aufgrund der daraus resultierenden weiteren Kompression der Federeinrichtung 16 wird deren Vorspannung weiter erhöht, was wiederum (aufgrund der Kraftumlenkung mittels der Kraftumlenkeinrichtung 18) zu einem zusätzlichen, in radialer Richtung auf das chirurgische Element 100 wirkenden Klemmkraftteil führt, sobald die Aufweitung der Klemmfinger 20 groß genug ist, um ein Passieren des chirurgischen Elements in Richtung auf die Fixiereinrichtung 28 zu ermöglichen. Nach dem vollständigen formschlüssigen Einführen in die Fixiereinrichtung 28 wird das chirurgische Element 100 nur durch Reibung im Bereich der Klemmfinger 20 in der Spannvorrichtung 10 gehalten.

Die axial gerichtete Federkraft der Federeinrichtung 16 wird also an den Klemmfingern 20 in eine radial nach innen wirkende Kraft durch das Zusammenwirken der Schrägfläche 24 der Kraftumlenkeinrichtung 18 mit den jeweiligen Klemmfingern 20 transformiert. Auf diese Weise wird das chirurgische Element 100 in der Spannvorrichtung 10 kraftschlüssig eingespannt. Aufgrund des Zusammenwirkens der Schrägfläche 24 unter Vorspannung mit den Klemmfingern 20 und dem davon gehaltenen chirurgischen Element 100 wird dieses ferner zentriert und spielfrei in der Spannvorrichtung 10 aufgenommen. Aus diesem Grund führt das chirurgische Element 100 keine Schlinger- oder Taumelbewegungen durch, wenn es in Drehung versetzt wird.

Ein weiterer Vorteil dieser Ausführung besteht darin, dass das chirurgische Element 100 ohne zusätzliche Mittel (Befestigungsschrauben, Hebel, usw.) eingespannt und wieder entfernt werden kann. Zusätzlich dient die Klemmeinrichtung 14 ausschließlich zur Fixierung und Zentrierung des eingespannten Elements 100, wohingegen die Übertragung von Drehbewegungen auf das chirurgische Element 100 durch die formschlüssige Verbindung Fixiereinrichtung 28 im Grundkörpers 12 erfolgt. Diese funktionale Trennung ermöglicht eine separate Optimierung der jeweiligen Funktionen.

Beispielsweise kann die Spannvorrichtung 10 abwechselnd Knochenschrauben mit 4 mm, 5 mm und 6 mm Durchmesser aufnehmen. Die Konzentration auf die Größen 4, 5 und 6 mm kommt daher, dass diese Schrauben (z.B. als so genannte Apex-Schrauben) in den Externen-Fixateur-Systemen der Anmelderin (Hoffmann II, Hoffmann Xpress, zukünftiges Hoffmann 3) verwendet werden können. Die Spannvorrichtung 10 muss dabei nur einmal am Werkzeug befestigt werden, danach ist außer dem Einschieben und Herausziehen der Knochenschrauben keinerlei Betätigung und insbesondere kein Wechsel der Spannvorrichtung 10 für Knochenschrauben mit unterschiedlichen Durchmessern mehr erforderlich.

Insgesamt führen die hier vorgeschlagenen Maßnahmen zu einer Reduktion der OP-Dauer, der Wahrscheinlichkeit von Fehlmanipulationen, der Wahrscheinlichkeit dass Knochenschrauben herunterfallen und der Verwechslungsgefahr von Kupplungen bzw. damit ausgestatten Spannvorrichtungen. Ferner ist eine einfache Demontage der Spannvorrichtung 10 möglich aufgrund der Schraubverbindung zwischen der Umlenkeinrichtung 18 und dem Grundkörper 12. Dies erleichtert die Sterilisation und Reinigung.

## Patentansprüche

1. Spannvorrichtung (10) für chirurgische Elemente (100), umfassend:
- eine Fixiereinrichtung (28), die zur drehfesten Fixierung eines chirurgischen Elements (100) ausgebildet ist;
- eine Klemmeinrichtung (14), die zur Ausübung einer im Wesentlichen radial nach innen auf das chirurgische Element (100) wirkenden Klemmkraft ausgebildet ist;
- eine Federeinrichtung (16), welche zur Ausübung einer Federkraft auf die Klemmeinrichtung (14) ausgebildet ist, wobei die Federkraft in axialer Richtung wirkt; und
- eine mit der Klemmeinrichtung (14) zusammenwirkenden Kraftumlenkeinrichtung (18), welche ausgebildet ist, zumindest einen Teil der auf die Klemmeinrichtung (14) wirkende Federkraft in zumindest einen Teil der auf das chirurgische Element (100) wirkenden Klemmkraft umzuwandeln, wobei die Klemmeinrichtung (14) wenigstens einen in radialer Richtung auslenkbaren Klemmfinger (20) umfasst.

2. Spannvorrichtung (10) nach Anspruch 1, wobei die Klemmeinrichtung (14) eine Kontaktierungsfläche für das chirurgische Element (100) aufweist, um bei Einführen des chirurgischen Elements (100) in die Spannvorrichtung (10) entgegen der Federkraft verschoben zu werden.

3. Spannvorrichtung (10) nach Anspruch 1, wobei mehrere Klemmfinger (20) vorhanden sind, welche das chirurgische Element (100) in dessen Umfangsrichtung umgeben.

4. Spannvorrichtung (10) nach Anspruch 2, wobei die Kontaktierungsfläche als Schrägfläche an dem wenigstens einen Klemmfinger (20) ausgebildet ist, um eine Kraftkomponente auf den Klemmfinger (20) radial nach außen zu erzeugen.

5. Spannvorrichtung (10) nach einem der vorhergehenden Ansprüche, wobei die Kraftumlenkeinrichtung (18) wenigstens eine erste Schrägfläche (24) umfasst, gegen welche die Klemmeinrichtung (14) mittels der Federeinrichtung (16) vorgespannt oder vorspannbar ist.

6. Spannvorrichtung (10) nach Anspruch 5, wobei die Klemmeinrichtung (14) wenigstens eine zweite Schrägfläche (26) umfasst, die mit der wenigstens einen ersten Schrägfläche (24) der Kraftumlenkeinrichtung (18) in kraftumlenkender Weise zusammenwirkt.

7. Spannvorrichtung (10) nach einem der vorhergehenden Ansprüche, wobei die Fixiereinrichtung (28) eine Öffnung zur Aufnahme eines Teils des chirurgischen Elements (100) aufweist, und wobei die Öffnung mit einer Profilierung versehen ist, die eine drehfeste Kopplung mit dem eine komplementäre Profilierung aufweisenden chirurgischen Element (100) gestattet.

8. Spannvorrichtung (10) nach Anspruch 7, wobei die Öffnung mehrere in axialer Richtung voneinander beabstandete und sich in distaler Richtung verjüngende Abschnitte zur drehfesten Aufnahme von chirurgischen Elementen (100) unterschiedlichen Außendurchmessers aufweist.

9. Spannvorrichtung (10) nach einem der vorhergehenden Ansprüche, ferner umfassend ein Gehäuseteil, welches die Klemmeinrichtung (14) außen wenigstens teilweise umgibt.

10. Spannvorrichtung (10) nach einem der vorhergehenden Ansprüche, ferner umfassend einen Grundkörper, in dem die Fixiereinrichtung ausgebildet ist, wobei am Grundkörper ein optionales Gegenlager für die Federeinrichtung ausgebildet ist.

11. Spannvorrichtung (10) nach den Ansprüchen 9 und 10, wobei das Gehäuseteil mit dem Grundkörper unter Gewährleistung einer vorgegebenen oder vorgebbaren axialen Relativposition gekoppelt ist.

12. Chirurgisches Werkzeug umfassend die Spannvorrichtung nach einem der Ansprüche 1 bis 11.

13. Chirurgisches System, umfassend das chirurgische Werkzeug nach Anspruch 12 sowie mehrere einzuspannende chirurgische Elemente mit unterschiedlichen Außendurchmessern.

14. Chirurgisches System nach Anspruch 13, ferner umfassend wenigstens ein Rahmenelement eines externen Fixateurs, wobei die chirurgischen Elemente zur Befestigung des wenigstens einen Rahmenelements an einem Knochen ausgebildet sind.

## Claims

1. A tensioning device (10) for surgical elements (100) comprising:
- a fixation device (28), designed for the torque-proof fixation of a surgical element (100);
- a clamping device (14), designed for the exertion of an essentially radial inward clamping force on the surgical element (100);
- a spring device (16), designed for the exertion of a spring force on the clamping device (14), wherein the spring force is exerted in an axial direction; and
- a force deflecting device (18) that interacts with the clamping device (14), which is designed to convert at least a part of the spring force exerted on the clamping device (14) into at least a part of the clamping force exerted on the surgical element (100), wherein the clamping device (14) comprises at least one clamping finger (20) that can be deflected in a radial direction.

2. The tensioning device (10) as claimed in Claim 1, wherein the clamping device (14) has a contact surface for the surgical element (100), such that when the surgical element (100) is inserted into the tensioning device (10), it can be moved against the spring force.

3. The tensioning device (10) as claimed in Claim 1, wherein a plurality of clamping fingers (20) are provided, which surround the surgical element (100) in its circumferential direction.

4. The tensioning device (10) as claimed in Claim 2, wherein the contact surface is designed as a slanted surface on the at least one clamping finger (20), in order to produce a force component on the clamping finger (20) radially outward.

5. The tensioning device (10) as claimed in one of the previous Claims, wherein the force deflecting device (18) comprises at least one first slanted surface (24), against which the clamping device (14) is or can be pre-tensioned by means of the spring device (16).

6. The tensioning device (10) as claimed in Claim 5, wherein the clamping device (14) comprises at least one second slanted surface (26), which interacts with the at least one first slanted surface (24) of the force deflecting device (18) in a force deflecting manner.

7. The tensioning device (10) as claimed in one of the previous Claims, wherein the fixation device (28) has an opening to receive a part of the surgical element (100), and wherein the opening is provided with a profile that allows a torque-proof coupling with the surgical element (100) having a complementary profile.

8. The tensioning device (10) as claimed in Claim 7, wherein the opening has several segments spaced at intervals from each other in an axial direction and tapering in a distal direction, for the torque-proof retaining of surgical elements (100) of different outer diameters.

9. The tensioning device (10) as claimed in one of the previous Claims, further comprising a casing part, which at least partially surrounds the clamping device (14) from the outside.

10. The tensioning device (10) as claimed in one of the previous Claims, further comprising a base body in which a fixation device is arranged, wherein at the base body an optional counter bearing for the spring device is formed.

11. The tensioning device (10) as claimed in Claim 9 and 10, wherein the casing part is coupled with the base body assuring a pre-set or settable axial relative position.

12. A surgical instrument comprising the tensioning device as claimed in one of the Claims 1 to 11.

13. A surgical system comprising the surgical instrument as claimed in Claim 12 and several surgical elements to be clamped, the surgical elements having different outer diameters.

14. The surgical system of Claim 13, further comprising at least one frame element of a fixateur externe, wheren the surgical elements are designed for fixation of the at least one frame element to a bone.

## Revendications

1. Dispositif de serrage (10) pour éléments chirurgicaux (100), comprenant :
- un moyen de fixation (28) conçu pour fixer de manière fixe en rotation un élément chirurgical (100) ;
- un moyen de serrage (14) conçu pour exercer une force de serrage agissant de manière sensiblement radiale vers l'intérieur sur l'élément chirurgical (100) ;
- un moyen formant ressort (16) conçu pour exercer une force élastique sur le moyen de serrage (14), la force élastique agissant dans la direction axiale ; et
- un moyen de renvoi de force (18) coopérant avec le moyen de serrage (14) et conçu pour convertir au moins une partie de la force élastique agissant sur le moyen de serrage (14) en au moins une partie de la force de serrage agissant sur l'élément chirurgical (100), le moyen de serrage (14) comportant au moins un doigt de serrage (20) pouvant être dévié dans la direction radiale.

2. Dispositif de serrage (10) selon la revendication 1, le moyen de serrage (14) présentant une surface de contact pour l'élément chirurgical (100) pour le déplacement dudit moyen de serrage contre la force exercée par le moyen formant ressort lorsque l'élément chirurgical est introduit dans le dispositif de serrage (10).

3. Dispositif de serrage (10) selon la revendication 1, plusieurs doigts de serrage (20), qui entourent l'élément chirurgical (100) sur sa circonférence, étant présents.

4. Dispositif de serrage (10) selon la revendication 2, la surface de contact étant conçue sous forme de surface inclinée sur ledit au moins un doigt de serrage (20) pour exercer de manière radiale vers l'extérieur une composante de force sur le doigt de serrage (20).

5. Dispositif de serrage (10) selon l'une des revendications précédentes, le moyen de renvoi de force (18) comportant au moins une première surface inclinée (24) contre laquelle le moyen de serrage (14) est ou peut être précontraint à l'aide du moyen formant ressort (16).

6. Dispositif de serrage (10) selon la revendication 5, le moyen de serrage (14) comportant au moins une deuxième surface inclinée (26) qui coopère avec ladite au moins une première surface inclinée (24) du moyen de renvoi de force (18) de manière à dévier la force en jeu.

7. Dispositif de serrage (10) selon l'une des revendications précédentes, le moyen de fixation (28) présentant une ouverture destinée à recevoir une partie de l'élément chirurgical (100), et ladite ouverture étant munie d'un profilage permettant un accouplement fixe en rotation avec l'élément chirurgical (100) présentant un profilage complémentaire.

8. Dispositif de serrage (10) selon la revendication 7, l'ouverture présentant plusieurs sections espacées les unes des autres dans la direction axiale et allant en se rétrécissant dans la direction distale pour le logement d'éléments chirurgicaux (100) de diamètres extérieurs différents.

9. Dispositif de serrage (10) selon l'une des revendications précédentes, comprenant en outre un élément de boîtier qui entoure extérieurement au moins partiellement le moyen de serrage (14).

10. Dispositif de serrage (10) selon l'une des revendications précédentes, comprenant en outre un corps de base dans lequel est réalisé le moyen de fixation, une contre-butée optionnelle pour le moyen formant ressort étant réalisée sur le corps de base.

11. Dispositif de serrage (10) selon les revendications 9 et 10, l'élément de boîtier étant accouplé avec le corps de base de manière à garantir une position axiale relative prédéfinie ou pouvant être prédéfinie.

12. Outil chirurgical comprenant le dispositif de fixation selon l'une des revendications 1 à 11.

13. Système chirurgical comprenant l'outil chirurgical selon la revendication 12 et plusieurs éléments chirurgicaux de diamètres extérieurs différents à fixer.

14. Système chirurgical selon la revendication 13, comprenant en outre au moins un élément cadre d'un fixateur externe, les éléments chirurgicaux étant conçus pour fixer ledit au moins un élément cadre sur un os.
